# EUROPEAN PATENT SPECIFICATION

(11) **EP 3 194 496 B1**
(45) Date of publication and mention of the grant of the patent: **25.11.2020**
(21) Application number: 15841782.4
(22) Date of filing: 08.09.2015
(51) Int. Cl.: C08K 5/04, C08K 5/1515, C08K 5/103, C08L 27/06, C08L 27/16, C08L 27/08, C07C 69/33, C11C 3/00, C11C 3/04

(54) **A HIGHLY STABILE PLASTICIZED POLYVINYL HALIDE COMPOSITION**
SEHR STABILE WEICHGEMACHTE POLYVINYLHALIDZUSAMMENSETZUNG
COMPOSITION D'HALOGÉNURE DE POLYVINYLE PLASTIFIÉE À STABILITÉ ÉLEVÉE

(30) Priority: 15.09.2014 SE 1400432
(43) Date of publication of application: 26.07.2017
(73) Proprietor: PERSTORP AB, 284 80 Perstorp (SE)
(72) Inventor: PAULSSON, Christoffer, 241 35 Eslöv (SE)
(74) Representative: Hoffmann Eitle
(86) International application number: PCT/SE2015/000053
(87) International publication number: WO 2016/043639

(56) References cited:
- EP-A2- 2 070 980
- WO-A1-01/00722
- WO-A1-2009/102877
- WO-A1-2011/041388
- WO-A1-2011/163434
- WO-A1-2012/026861
- WO-A1-2014/084313
- DE-A1- 1 618 586
- US-A- 3 670 013
- US-A- 4 421 886
- US-A- 5 036 121
- US-A1- 2011 272 174
- SHOBHA, H. K. ET AL.: 'Structural expressions of long-chain esters on their plasticizing behavior in poly(vinyl chloride' MARCOMOLECULES vol. 25, no. 25, 1992, pages 6765 - 6769, XP055147694
- KISHORE, K. ET AL.: 'Plasticization phenomena and fire retardancy in PVC' INDIAN JOURNAL OF CHEMISTRY, SECTION A vol. 31 A, no. 8, 1992, pages 590 - 595, XP008172846

## Description

The present invention refers to a highly stable plasticised polyvinyl halide composition, said composition comprising polyvinylhalide polymer, pentaerythrithol tetravalerate and epoxidized unsaturated fatty acid ester.

Plasticisers have the ability to reduce the glass transition temperature of polymers and thereby provide soft and/or flexible products, contrary to the hard and brittle basic material. Organic esters constitute the major group of plasticisers for polyvinylhalide polymers such as polyvinylchloride (PVC), polyvinylidenechloride (PVDC) and polyvinylideneflouride (PVF2). Among these organic esters phthalates are without competition the largest subgroup. The most commonly used phthalate esters as PVC plasticisers are di-2-ethylhexyl phthalate (DEHP), also known as dioctyl phthalate (DOP), diisononyl phthalate (DINP) and diisodecyl phthalate (DIDP). Further examples of organic esters used as PVC plasticisers are adipates, trimellitates, sebacates and azelates. Phosphate esters, such as tris (2-ethylhexyl) phosphate, and sulphonate esters, such as arylesters of sulphonic acids, are used in minor amounts. The preparation and the use of said plasticisers are well known and thoroughly disclosed in a number of handbooks and encyclopedias of chemical technology.

WO 01/00722 A1 discloses plasticisers for polymeric composition such as poly(vinyl halides) and poly(vinylidene halides) which plasticisers comprise at least one polyolester built up from a 2-alykl-2-hydroxyalkyl-1,3-propanediol or a 2,2,-di(hydroxyalkyl)-1,3-propanediol or from a dimer, a trimer or a polymer thereof. The plastisol of the poly(vinyl halides) or poly(vinylidene halide) may also further contain epoxidized soybean oil.

Plasticized polyvinylhalides is used in a multitude of plastic items going to a multitude of end-user sectors such as flexible films, cables and wires, flooring, flexible tubes and profiles and coated fabric and paper. Over the last few decades, the use of plastics as packaging materials has increased due to their properties and processability. As a result of contact between packed food and plastics, traces of plasticisers may migrate into the food contaminating it and affecting consumers' health. The situation has created an increasing desire for phthalate-free plasticisers for applications like toys, food contact materials, medicals and other applications where the use of phthalates are either restricted or non-wanted by end-users.

Another problem with plasticized polyvinylhalides is the useful product life due to stability of the composition itself. Some of these problems relate to the plasticizer per se while others relate to compatibility between the polymer and plasticizer. These stability problems can be separated into the three categories; thermal-, ultraviolet- (UV) and hydrolyzation- stability. The thermal stability is particularly important in respect of processability of the plasticized polymer such as when agglomerating the polymer/plasticizer mixture but also when injection molding articles of said plasticized polymer.

Long term thermal stability is also an important consideration in articles that would be subjected to elevated temperatures over a long time. As an example of such articles can be mentioned hoses connecting the radiator of a vehicle to the engine. The hot water may here cause migration of plasticizer. It may also cause hydrolyzation of the plasticizer itself. In the long run this will cause the hose in this specific example to become brittle. Eventually cracks and leaks will appear.

Most plasticized polyvinylhalides have a poor stability towards UV light. This will be noticed by yellowing, the surface becoming sticky and slippery, the plasticized polymer becoming harder and more brittle and in some cases shrinking. This will radically reduce the useful life of plasticized polyvinylhalides in outdoor use.

Accordingly, the invention relates to a highly stabile plasticized polyvinylhalide composition as defined in the appended claims. The invention is characterized in that the composition comprises polyvinylhalide, pentaerythritol tetravalerate and an epoxidized unsaturated fatty acid ester having an iodine value of at least 135 prior to the epoxidation. This means that an unsaturated fatty acid ester having an iodine value of at least 135 is epoxidized. The iodine value will suitably be below 25, preferably below 10 after epoxidation.

According to the invention the epoxidized unsaturated fatty acid ester has at least five epoxide groups.

The polyvinylhalide is suitably selected from the group consisting of polyvinylchloride, polyvinylidenedichloride, polyvinylidenediflouride.

According to the invention the epoxidized unsaturated fatty acid ester is a glycerol ester selected from the group consisting of linseed oil and tung oil. Said linseed oil and/or tung oil is accordingly epoxidized so that the average ester molecule comprises five or more epoxide groups.

According to the invention a plasticizer-mixture of 2 - 10% by weight of the epoxidized unsaturated fatty acid ester and balance to 100% by weight of pentaerythritol tetravalerate is present in the highly stabile plasticized polyvinylhalide composition. The highly stabile plasticized polyvinylhalide composition suitable comprises 10 - 60 % by weight of plasticizer-mixture.

According to a special embodiment of the invention, the highly stabile plasticized polyvinylhalide composition further comprises a PVC stabilizer selected from the group consisting of barium/zink compound and calcium/zink compound. The purpose of such a PVC stabilizer is foremost to stabilize the polymer in the composition according to the invention. The PVC stabilizer may further comprise a co-stabilizer containing polyhydric alcohols selected from the group consisting of mono-pentaerythritol, di-pentaerythritol, trimethylolpropane, di-trimethylolpropane, pentaerythritol adipate and mixtures thereof.

The present invention is further explained with reference to enclosed embodiment Examples, which are to be construed as illustrative and not limiting in any way.
Example 1a illustrates evaluation of the UV stability of plasticized PVC compositions.
Example 1b illustrates evaluation of the Thermal stability of plasticized PVC compositions.
Example 1c illustrates evaluation of the hydrolytic stability of plasticized PVC compositions.

### Example 1a

A number of samples were prepared as a first trial to evaluate the effect of UV light.

Sheets having a thickness of 80 - 100µm were prepared. These sample sheets consisted of the following compositions;

| Sample No. | PVC K-value 70 | Plasticizer /amount | Stabilizer /amount |
|---|---|---|---|
| I | 100 phr | Pentaerythrithol tetravalerate / 30 phr | Ba/Zn / 2 phr |
| II | 100 phr | Diisononyl phthalate / 30 phr | Ba/Zn / 2 phr |
| III | 100 phr | Diisononyl cyclohexane-1,2-dicarboxylate / 30 phr | Ba/Zn / 2 phr |
| IV | 100 phr | Dioctyl phthalate / 30 phr | Ba/Zn / 2 phr |
| V | 100 phr | Dioctyl terephthalate / 30 phr | Ba/Zn / 2 phr |

The samples were subjected to a QUV test for 1750 hours. The test were run in cycles of 4h UV-A light at 60°C and 4h conditioning at 50°C in accordance with ISO 4891-1 and ISO 4892-2. The tests were performed in a QUV Weathering tester from Q-Tester.

The yellowing index Yi was determined in accordance with ASTM D 1925-70 at 24 hours, 240 hours, 500 hours and then at 250 hour interval until 1750 hours was reached.

The following results were obtained;

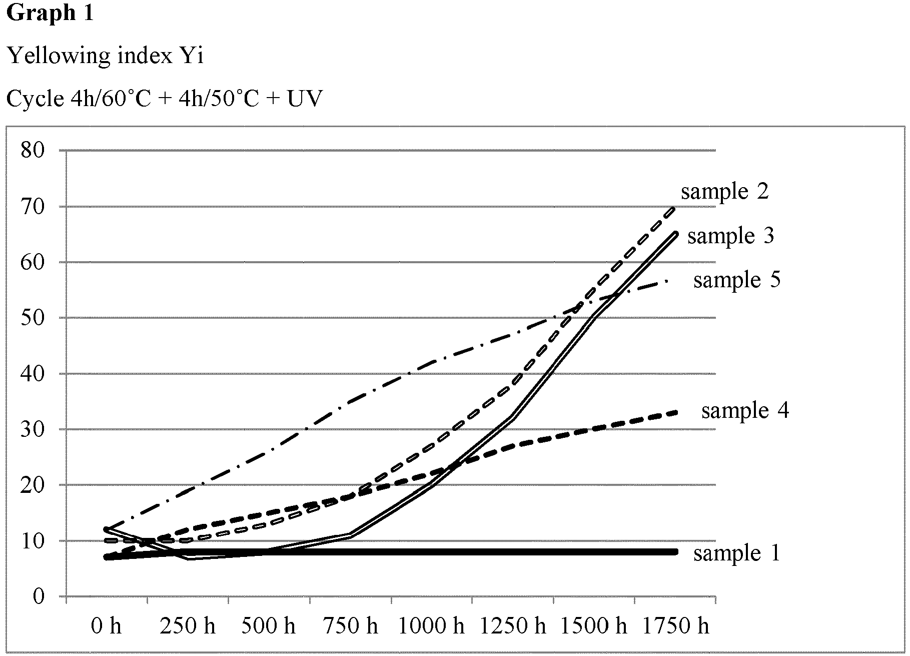

The conclusion of the trial is that sample 1, a PVC resin plasticized with pentaerythrithol tetravalerate is virtually unaffected while the other samples show varying degree of increasing yellowing over the test period.

There was accordingly no reason to perform testing on thermal and hydrolytic stability on sample 2 - 5 as they did not perform well in the UV test.

### Example 1b

A set of samples was prepared as to evaluate the thermal stability.

Sheets having a thickness of 1mm were prepared. These sample sheets consisted of the following compositions;

| Sample No. | Polymer /amount | Plasticizer /amount | Co-plasticizer /amount | Stabilizer /amount |
|---|---|---|---|---|
| VI | PVC / 100 phr | Pentaerythrithol-tetravalerate / 70 phr | Lancroflex L / 1 phr | Ca/Zn / 2 phr |
| VII | PVC / 100 phr | Pentaerythrithol-tetravalerate / 70 phr | Lancroflex L / 1.5 phr | Ca/Zn / 2 phr |

The samples were subjected to 200°C for 2 minutes in a Mathis oven. Thermal stability was measured with a Werner-Mathis oven thermo tester. The films were subjected to 200°C in the oven. After 5 min ground time the films were removed from the oven in 20 mm steps every 60 s. The time until the film became discoloured was measured for each film.

It showed that sample VI and VII had sufficient stability for normal processing with the ability to endure the temperature without coloration for 12 - 15 minutes. As comparison commercially available compositions II and III typically will endure the same temperature for 10 - 12 minutes.

### Example 1c

A set of samples was prepared as to evaluate the hydrolytic stability.

The samples correspond fully to sample VI and VII in example 1b above.

The samples were subjected to 80°C at a relative humidity of 100% in a climate chamber for 1 week. The evaporation of valeric acid, indicating hydrolysation of the pentaerythrithol tetravalerate was measured. The amount of valeric acid detected was below 5 ppm for both samples indicating that the compositions are very stable.

We find our conclusions to be that a plasticized polyvinyl halide composition in accordance to the invention is indeed very stable and surprisingly stabile in outdoor applications due to its ability to withstand UV radiation without noticeable degradation of the composition.

### Example 2a

Once the thermal, hydrolytic and UV stability was analysed, a further series of trials were performed in order to analyse and compare other important characteristics of plasticized PVC. It was considered that the characteristics; fusion time, Shore hardness, migration and volatility would not be greatly affected by the co-plasticizer whereby this was omitted for the sake of simplicity.

### Preparation of plasticised PVC sheets

PVC resins (suspensions of PVC particles in a plasticiser) of below formulations were prepared:

| Sample No. | Polymer (Norvinyl S-706) / (g) | Plasticizer / (g) | Stabilizer / (g) |
|---|---|---|---|
| VIII | PVC / 190.8 | Pentaerythrithol tetravalerate / 95.4 | Ba/Zn / 3.8 |
| IX | PVC / 190.8 | Diisononyl phthalate / 95.4 | Ba/Zn / 3.8 |
| X | PVC / 190.8 | Diisononyl cyclohexane-1,2-dicarboxylate / 95.4 | Ba/Zn / 3.8 |
| XI | PVC / 190.8 | Dioctyl phthalate / 95.4 | Ba/Zn / 3.8 |

The components of each PVC resin were carefully mixed and then calendared to a sheet using a two-roll mill at 165°C.

### Example 2b

### Evaluation of the obtained plasticised PVC sheets

All plasticised PVC sheets obtained in Example 4 were evaluated regarding fusion time, hardness, migration and volatility.

### Fusion Time

A test to determine the time required for PVC and plasticiser to completely mix together and form a uniform blend. A mixing bowl was heated to the test temperature of 88°C and charged with 300 g PVC resin. A stirring of 60 rpm was applied for 5 minutes to allow the resin to reach the bowl temperature. 150 g plasticizer was then added and the time required for PVC and plasticiser to completely mix together and form a uniform blend was measured.

The results are presented in Chart 1.

### Hardness

### Durometer hardness Shore A. (Standard: ASTM 2240:3)

A test based on the penetration of a specific type of indentor when forced into the material under specific conditions. The indentation hardness is inversely related to the penetration and dependant on the elastic modulus and viscoelastic behaviour of the material.

The results are presented in Chart 2.

### Migration

### Determination of migration of plasticisers (Standard: ISO 177)

A test based on quantitative determination of the loss of mass of a sheet of plasticized plastic placed between two fresh absorbent backing discs. A rubber-PVC-rubber sandwich was wrapped with aluminium foil and rubber sheets before being placed between two glass plates. A weight of 5kg was placed on the sandwich assembly and the whole package was placed in an oven with a temperature of 70 ±2°C. The samples were then picked out and the weight of both the plasticized plastic and the absorbent backing discs was measured after 3, 7, 14 and 28 days.

The results for 28 days are presented in Chart 3.

### Volatility

### Activated carbon method (Standard: ISO 176 Method A)

A test method based on quantitative determination of the loss of plasticiser from plasticized plastic materials upon heating, where it is generally assumed that no significant amounts of other volatile materials are present. 120cm³ of activated carbon was spread on the bottom of metal container and a test sample was placed on top of the carbon and covered with another 120cm³ of activated carbon. Two further samples were placed in the container, each covered with 120cm³ of carbon, where after the container was sealed with a lid. The container was placed in oven with a temperature of 100±1°C. The activated carbon surrounding the samples absorbed the plasticiser extracted upon heating. After 7 days the container was removed from the oven and cooled to room temperature. The samples were removed from the container, carefully brushed free from carbon particles and weighed.

The results are presented in Chart 4.

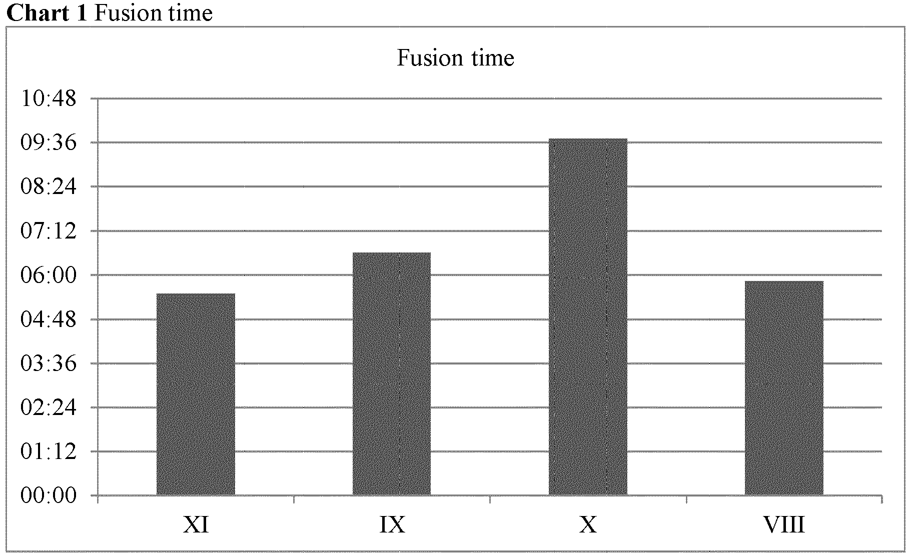

The fusion time for the plasticiser VIII according to the present invention was found to be shorter than plasticisers IX and X.

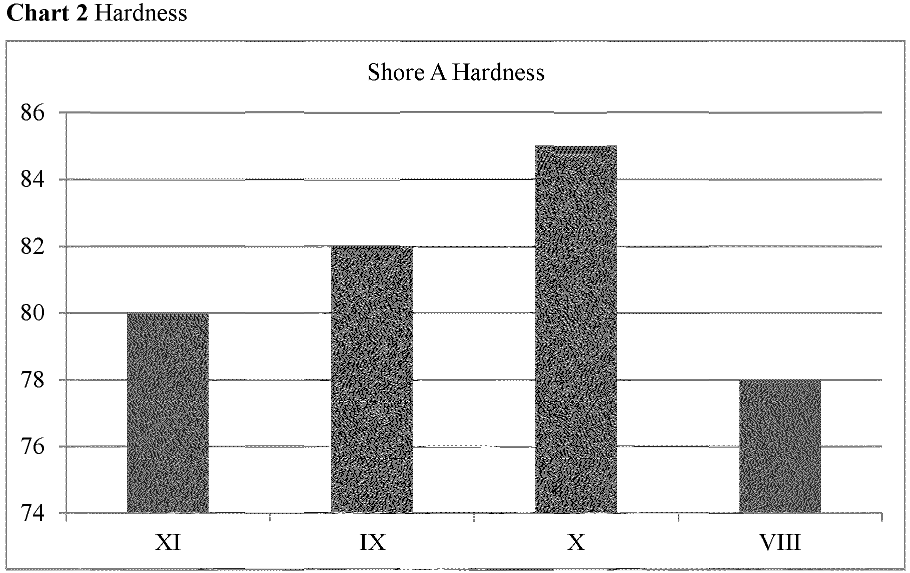

The plasticising effect of the plasticisers according to the present invention was found to be high in comparison to commercially available plasticisers XI, IX and X. This means that lower amounts of plasticiser can be added with maintained hardness.

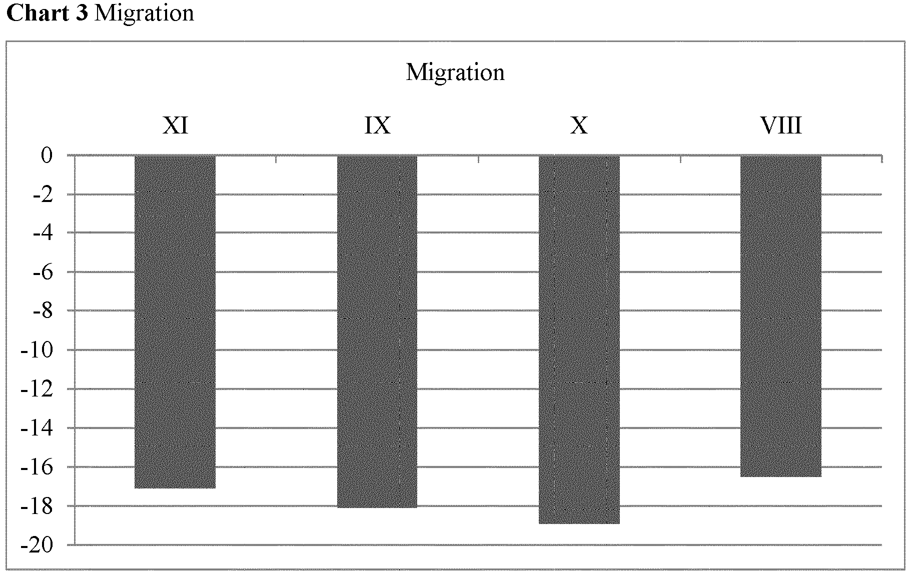

The plasticiser VIII according to the invention showed very good migration values.

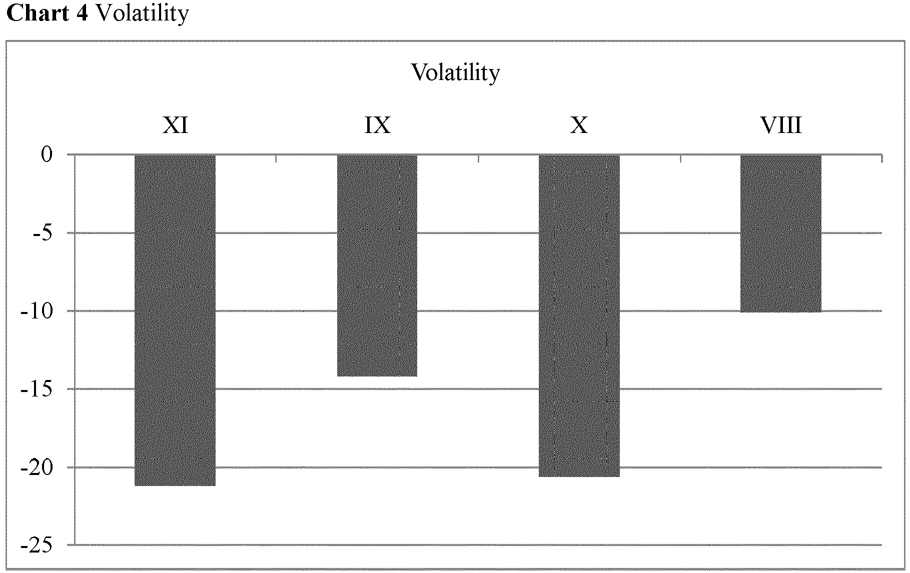

The plasticiser VIII according to the invention showed very good volatility value. The comparative example XI and X showed to be rather poor due to its relatively high volatility.

It has, during experimentation with the plasticisers according to the invention been found that the fusion time is very short, while quite surprisingly, the volatility is lower than commercially available plasticisers. The short fusion time implies that the processing time can be shorter and/or temperature during agglomeration of plasticiser can be lower wherein any problem with thermal stability during processing of the composition according to the invention becomes even less significant.

The composition in accordance with the invention has also been found to be remarkably UV-stabile as well as having fully sufficient thermal and hydrolytic stability. It also has the advantage of not containing phthalate based plasticisers, which is a desired property. The low volatility together with the high level of UV-stability will increase the useful life of products made of a composition in accordance with the invention.

## Claims

1. A highly stabile plasticized polyvinylhalide composition, **characterized in that** the composition comprises polyvinylhalide, pentaerythritol tetravalerate and an epoxidized unsaturated fatty acid ester, the unsaturated fatty acid ester having at least five epoxide groups and an iodine value of at least 135 prior to epoxidation and is a glycerol ester selected from the group consisting of linseed oil and tung oil, wherein a plasticizer-mixture of 2 - 10% by weight of the epoxidized unsaturated fatty acid ester and balance to 100% by weight of pentaerythritol tetravalerate is present in the highly stabile plasticized polyvinylhalide composition.

2. A composition according to claim 1, **characterized in that** the polyvinylhalide is selected from the group consisting of polyvinylchloride, polyvinylidenechloride, polyvinylidenefluoride

3. A composition according to claim 1 or 2, **characterized in that** the highly stabile plasticized polyvinylhalide composition comprises 10 - 60 % by weight of plasticizer-mixture.

4. A composition according to any of claims 1-3, **characterized in that** the highly stabile plasticized polyvinylhalide composition further comprises a PVC stabilizer selected from the group consisting of barium/zink compound and calcium/zink compound.

5. A composition according to claim 4, **characterized in** the PVC stabilizer further comprises a co-stabilizer containing polyhydric alcohols selected from the group consisting of mono-pentaerythritol, di-pentaerythritol, trimethylolpropane, di-trimethylolpropane and mixtures thereof.

## Patentansprüche

1. Hochstabile weichgemachte Polyvinylhalogenidzusammensetzung, **dadurch gekennzeichnet, dass** die Zusammensetzung Polyvinylhalogenid, Pentaerythritoltetravalerat und einen epoxidierten ungesättigten Fettsäureester umfasst, wobei der ungesättigte Fettsäureester mindestens fünf Epoxidgruppen und vor der Epoxidierung einen Iodwert von mindestens 135 aufweist und ein Glycerolester ist, ausgewählt aus der Gruppe, bestehend aus Leinöl und Tungöl, worin in der hochstabilen weichgemachten Polyvinylhalogenidzusammensetzung ein Weichmachergemisch aus 2-10 Gew.-% des epoxidierten ungesättigten Fettsäureesters und einem Rest zu 100 Gew.-% von Pentaerythritoltetravalerat vorhanden ist.

2. Zusammensetzung gemäß Anspruch 1, **dadurch gekennzeichnet, dass** das Polyvinylhalogenid aus der Gruppe ausgewählt ist, bestehend aus Polyvinylchlorid, Polyvinylidenchlorid, Polyvinylidenfluoride.

3. Zusammensetzung gemäß Anspruch 1 oder 2, **dadurch gekennzeichnet, dass** die hochstabile weichgemachte Polyvinylhalogenidzusammensetzung 10-60 Gew.-% des Weichmachergemischs umfasst.

4. Zusammensetzung gemäß mindestens einem der Ansprüche 1-3, **dadurch gekennzeichnet, dass** die hochstabile weichgemachte Polyvinylhalogenidzusammensetzung ferner einen PVC-Stabilisator umfasst, ausgewählt aus der Gruppe, bestehend aus einer Barium-/Zinkverbindung und einer Calcium-/Zinkverbindung.

5. Zusammensetzung gemäß Anspruch 4, **dadurch gekennzeichnet, dass** der PVC-Stabilisator ferner einen Co-Stabilisator umfasst, der mehrwertige Alkohole enthält, ausgewählt aus der Gruppe, bestehend aus Monopentaerythritol, Dipentaerythritol, Trimethylolpropan, Ditrimethylolpropan und Gemischen davon.

## Revendications

1. Composition d'halogénure de polyvinyle plastifié à haute stabilité, **caractérisée en ce que** la composition comprend de l'halogénure de polyvinyle, un tétravalérate de pentaérythritol et un ester d'acide gras insaturé epoxydé, l'ester d'acide gras insaturé ayant au moins cinq groupes époxydes et un valeur d'iode d'au moins 135 avant l'époxydation et est un ester de glycérol choisi parmi le groupe constitué de l'huile de lin et l'huile de tung, dans laquelle un mélange de plastifiant de 2 - 10% en poids de l'ester d'acide gras insaturé epoxydé et un reste à 100% en poids de tétravalérate de pentaérythritol est présent dans la composition d'halogénure de polyvinyle plastifiée à haute stabilité.

2. Composition selon la revendication 1, **caractérisée en ce que** l'halogénure de polyvinyle est choisi parmi le groupe constitué du chlorure de polyvinyle, du chlorure de polyvinylidène, du fluorure de polyvinylidène.

3. Composition selon la revendication 1 ou 2, **caractérisée en ce que** la composition d'halogénure de polyvinyle plastifiée à haute stabilité comprend de 10 - 60 % en poids du mélange de plastifiant.

4. Composition selon l'une quelconque des revendications 1-3, **caractérisée en ce que** la composition d'halogénure de polyvinyle plastifiée à haute stabilité comprend en outre un agent de stabilisation PVC choisi parmi le groupe constitué d'un composé baryum/zinc et d'un composé calcium/zinc.

5. Composition selon la revendication 4, **caractérisée en ce que** l'agent de stabilisation PVC comprend un co-agent de stabilisation contenant des alcools polyhydriques choisis parmi le groupe constitué du mono-pentaérythritol, du di-pentaérythritol, du triméthylolpropane, du di-triméthylolpropane et des mélanges de ceux-ci.
